# EUROPEAN PATENT APPLICATION

(11) **EP 4 563 085 A1**
(43) Date of publication of application: **04.06.2025**
(21) Application number: 24215544.8
(22) Date of filing: 26.11.2024
(51) Int. Cl.: A61B 5/16, A61B 5/00

(54) **ARABIC LANGUAGE EYE-TRACKING PARADIGM FOR THE EARLY SCREENING AND DIAGNOSIS OF AUTISM SPECTRUM DISORDERS**

(30) Priority: 29.11.2023 US 202363604125 P
(71) Applicant: Qatar Foundation for Education, Science and Community Development, Doha (QA)
(72) Inventor: Al-Shaban, Fouad A., Doha (QA)
(74) Representative: K&L Gates LLP

(57) **Abstract**

A system for screening and diagnosis of autism spectrum disorders using Arabic language eyetracking paradigm including a computing device, a screen device, an eye tracking device, and a set of stimuli paradigm. The set of stimuli paradigm are each comprised of dynamic individual faces, static side-by-side faces, joint attention bids, gaze following, reciprocal interactions, dynamic social versus dynamic geometric images, and passive viewing of social or object arrays. The set of stimuli paradigm each end a short gaze accuracy validation step. Eye tracking information is used to generate an Autism Index (AI) score.

## Description

### PRIORITY CLAIM AND CROSS-REFERENCE TO RELATED APPLICATIONS

The present application claims the benefit of U.S. Provisional Application No. 63/604,125 filed November 29, 2023, which is incorporated herein by reference in its entirety.

### FIELD

The present disclosure relates generally to a process and method for the early screening and diagnosis of autism spectrum disorders using an Arabic language eye-tracking paradigm.

### BACKGROUND

Abnormal eye gaze is a hallmark characteristic of Autism Spectrum Disorder (ASD). Social attention is a key developmental parameter that likely influences a range of developmental processes, including social cognition. Numerous studies have identified gaze differences between individuals with autism spectrum disorder (ASD) and controls across a wide range of ages and stimulus paradigms. Reductions in eye gaze to social stimuli and increases in attention to non-social stimuli are a replicable and relatively stable feature of ASD, consistent with original conceptualizations of autism, and is considered a red flag in all diagnostic instruments.

More than a decade of research into eye gaze abnormalities has confirmed social attention deficits as a main feature of ASD, even in very young children. Across studies, various stimulus patterns have induced social attention abnormalities; ranging from diminished fixation to others' eyes and social scenes as early as 6 months of age, to gaze abnormalities during dyadic or joint attention display in preschoolers and older children, to abnormal gaze towards dynamic social stimuli in older high-functioning individuals.

Presently, ASD is identified in the context of a clinical evaluation that is typically based on some combination of parent-report, parent-interview, and clinical observation tools. These methods are heavily influenced by subjective perceptions and require substantial training and ongoing inter-rater reliability checks. Several diagnostic tools have been validated as a "gold-standard" to enable clinicians to diagnose ASD and to differentiate it from other neurodevelopmental disorders, Like the Autism Diagnostic Observation Schedule-2^{nd} edition (ADOS-2) which is widely considered as one of the most effective evaluation tools for clinical and research contexts. Nonetheless, to date, all measures proven effective in diagnosing ASD are subjective and need extensive training and ongoing reliability checks to maintain accuracy and fidelity. None of the present diagnostic procedures include an objective marker that provides immediate interval-scale measurements and highly reliable scores across the full range of behaviors in individuals affected with ASD. Thus, development of objective measures of ASD has the potential to greatly enhance clinical evaluation, supplementing existing subjective assessment methods.

Recently, research has been geared towards investigations of objective markers for ASD diagnosis. Technological advancements such as eye gaze tracking have shown promise as a method for producing objective markers for ASD. Recent studies have provided support for the potential distinctive value of eye gaze tracking. In these studies, individual social stimuli had moderate and potentially informative discriminative value in distinguishing individuals with ASD and other developmental delays from typically developed healthy control individuals.

The development of a quantitative, interval-scale measure of autism symptoms, including measures of the core symptom domains, would represent a major step forward in the technology used to capture autism symptom levels and identify cases that are at risk for ASD diagnosis. Most recently, researchers showed that eye gaze to social and non-social stimuli can be aggregated into an Autism Index (AI) score with high validity for identification of ASD. This work has also shown that social attention processes are a distinct behavioral dimension that shows cross-cultural consistency and that remains stable throughout various age groups, with females exhibiting a slightly stronger preference, on average, for social attention. Thus, it remains possible that recent advances in the development of social attention-based metrics for assessing the likelihood of ASD might be transportable across cultural contexts after appropriate adaptation.

While social attention has been found to be consistent across cultures, and the AI has shown promise in identifying ASD in English-speaking populations, it is crucial to determine whether the tool can be successfully implemented in other contexts through further cross-cultural validation studies. This will expand the diagnostic tools available for ASD screening and improve access to accurate diagnosis for individuals from diverse backgrounds.

Therefore, there is a need to build on prior research in a US population to create a highly similar Arabic-language stimulus battery for collecting and scoring the AI. The stimuli need to be appropriate for the Middle Eastern culture, well accepted by parents, and maintain the child's attention during data collection. Relatedly, there is a need to compute the Autism Risk ("Autism Index" - AI) by aggregating gaze metrics across the new Arabic-language stimuli in an identical fashion to the prior US work, and validate the Arabic AI within the Qatari population through recruitment and collection of eye tracking data of a large sample of ASD affected individuals and Non-Autistic (NA)) children and children with other Developmental Delays (DD) between the ages of 3-15 years.

### SUMMARY

Example systems, methods, and apparatus are disclosed herein for development and validation of an Arabic language eye-tracking paradigm for the early screening and diagnosis of autism spectrum disorders in Qatar.

In light of the disclosure herein, and without limiting the scope of the invention in any way, in a first aspect of the present disclosure, which may be combined with any other aspect listed herein unless specified otherwise, a system for screening and diagnosis of autism spectrum disorders using Arabic language eye-tracking paradigm including a computing device, a screen device, an eye tracking device, and a set of stimuli paradigm. The set of stimuli paradigm are each comprised of dynamic individual faces, static side-by-side faces, j oint attention bids, gaze following, reciprocal interactions, dynamic social versus dynamic geometric images, and passive viewing of social or object arrays. The set of stimuli paradigm each end a short gaze accuracy validation step.

In a second aspect of the present disclosure, which may be combined with any other aspect listed herein unless specified otherwise, the computing device is operatively coupled to the screen device and the eye tracking device.

In a third aspect of the present disclosure, which may be combined with any other aspect listed herein unless specified otherwise, the eye tracker is operatively coupled to the screen device, such that the eye tracker tracks individual eye gaze information for an individual looking at the screen device.

In a fourth aspect of the present disclosure, which may be combined with any other aspect listed herein unless specified otherwise, the individual eye gaze information tracked is comprised of binocular gaze, 3D eye position, pupil, and timestamp data collected at a sampling rate of 250Hz. The individual eye gaze information tracked is calibrated using 2-, 5-, or 9-point calibrations starting with 5 or 9-point depending on a functional level of the individual looking at the screen device. The individual eye gaze information tracked is provided with position accuracy to 0.4°.

In a fifth aspect of the present disclosure, which may be combined with any other aspect listed herein unless specified otherwise, the computing device is configured to play one of the set of stimuli paradigms.

In a sixth aspect of the present disclosure, which may be combined with any other aspect listed herein unless specified otherwise, the computing device is configured to calculate dwell proportion based on the individual eye gaze information tracked. Dwell proportion is a percentage of the on-target region relative to the total time on the screen of one of the set of stimuli paradigms played. The computing device is configured to collect information on visits to regions-of-interest, saccade average length, saccade peak velocity, and blink frequency based on the individual eye gaze information tracked.

In a seventh aspect of the present disclosure, which may be combined with any other aspect listed herein unless specified otherwise, the computing device is configured to calculate an Autism Index (AI) score.

In an eighth aspect of the present disclosure, which may be combined with any other aspect listed herein unless specified otherwise, the set of stimuli paradigm is comprised of a first module and a second module.

In a ninth aspect of the present disclosure, which may be combined with any other aspect listed herein unless specified otherwise, the first module is comprised of 21 different photo and short video stimuli entirely in Arabic or entirely in English and is configured for use with nonverbal individuals and very young children.

In a tenth aspect of the present disclosure, which may be combined with any other aspect listed herein unless specified otherwise, the second module is comprised of 24 different photo and short video stimuli entirely in Arabic or entirely in English and the second module is configured for use with verbal individuals.

In an eleventh aspect of the present disclosure, which may be combined with any other aspect listed herein unless specified otherwise, the eye tracking device is one of a Tobbi or other eye tracker system.

In a twelfth aspect of the present disclosure, which may be combined with any other aspect listed herein unless specified otherwise, a method of using a system for screening and diagnosis of autism spectrum disorders using Arabic language eye-tracking paradigm comprising a computing device, a screen device, an eye tracking device and a set of stimuli paradigm. The set of stimuli paradigms is each comprised of dynamic individual faces, static side-by-side faces, joint attention bids, gaze following, reciprocal interactions, dynamic social versus dynamic geometric images, and passive viewing of social or object arrays. The set of stimuli paradigm each ends a short gaze accuracy validation step, the method includes playing one of the set of stimuli on the screen device, tracking individual eye gaze information for an individual looking at the screen device, and calculating an Autism Index (AI) score based on the individual eye gaze information.

In a thirteenth aspect of the present disclosure, which may be combined with any other aspect listed herein unless specified otherwise, the computing device is operatively coupled to the screen device and the eye tracking device, and the eye tracker is operatively coupled to the screen device, such that the eye tracker is configured to track individual eye gaze information for the individual looking at the screen device.

In a fourteenth aspect of the present disclosure, which may be combined with any other aspect listed herein unless specified otherwise, the individual eye gaze information tracked is comprised of binocular gaze, 3D eye position, pupil, and timestamp data collected at a sampling rate of 250Hz. The individual eye gaze information tracked is calibrated using 2-, 5-, or 9-point calibrations starting with 5 or 9-point depending on the functional level of the individual looking at the screen device. The individual eye gaze information tracked is provided with a position accuracy to 0.4°.

In a fifteenth aspect of the present disclosure, which may be combined with any other aspect listed herein unless specified otherwise, the computing device is configured to calculate dwell proportion based on the individual eye gaze information tracked. Dwell proportion is a percentage of the on-target region relative to the total time on the screen of one of the sets of stimuli paradigm played. The computing device is configured to collect information on visits to regions-of-interest, saccade average length, saccade peak velocity, and blink frequency based on the individual eye gaze information tracked.

In a sixteenth aspect of the present disclosure, which may be combined with any other aspect listed herein unless specified otherwise, the set of stimuli paradigm is comprised of a first module and a second module.

In a seventeenth aspect of the present disclosure, which may be combined with any other aspect listed herein unless specified otherwise, the first module is comprised of 21 different photo and short video stimuli entirely in Arabic or entirely in English and is configured for use with nonverbal individuals and very young children. The second module is comprised of 24 different photo and short video stimuli entirely in Arabic or entirely in English and is configured for use with verbal individuals.

In an eighteenth aspect of the present disclosure, which may be combined with any other aspect listed herein unless specified otherwise, the individual looking at the screen device is located about 60 to 65 cm from the screen device and looks at the screen device at a visual angle of about 18.8 degrees.

In a nineteenth aspect of the present disclosure, which may be combined with any other aspect listed herein unless specified otherwise, the individual eye gaze information tracked is tracked for 3-5 minutes.

In a twentieth aspect of the present disclosure, which may be combined with any other aspect listed herein unless specified otherwise, a method of calculating an Autism Index (AI) score, the method includes collecting individual eye gaze information for an individual looking at one of a set of stimuli paradigm, collecting information on visits to regions-of-interest, saccade average length, saccade peak velocity, and blink frequency based on the individual eye gaze information, and calculating a dwell proportion based on the individual eye gaze information. The set of stimuli paradigm is comprised of a first module and a second module, where the first module is comprised of 21 different photo and short video stimuli entirely in Arabic or entirely in English and is configured for use with nonverbal individuals and very young children. The second module is comprised of 24 different photo and short video stimuli entirely in Arabic or entirely in English and is configured for use with verbal individuals. The individual eye gaze information tracked is comprised of binocular gaze, 3D eye position, pupil, and timestamp data collected at a sampling rate of 250Hz. The individual eye gaze information tracked is calibrated using 2-, 5-, or 9-point calibrations starting with 5 or 9-point depending on the functional level of the individual looking at the screen device. The individual eye gaze information tracked is provided with a position accuracy of 0.4°. Dwell proportion is a percentage of on target region relative to the total time on the screen of one of the sets of stimuli paradigm played.

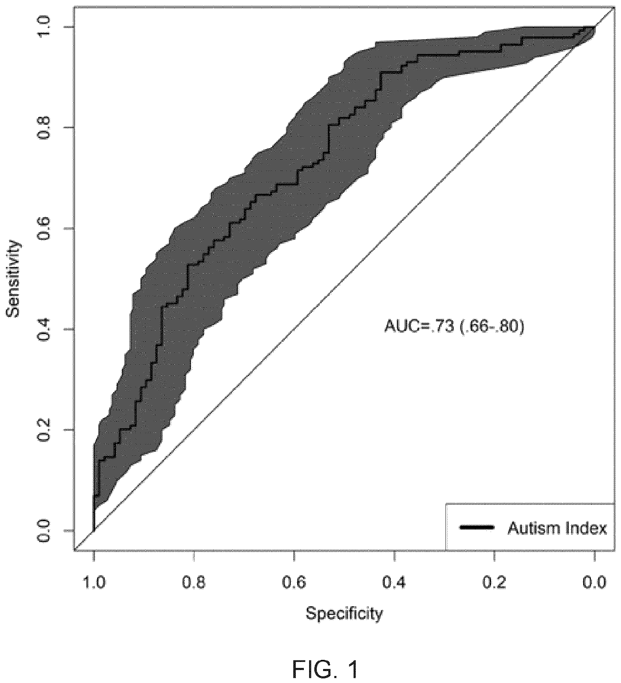

In the twenty-first aspect of the present disclosure, any of the structure, functionality, and alternatives disclosed in connection with any one or more of the Figures. 1 to 5 may be combined with any other structure, functionality, and alternatives disclosed in connection with any other one or more of the Figures. 1 to 5.

In light of the present disclosure and the above aspects, it is, therefore, an advantage of the present disclosure to provide users with an Arabic language eye-tracking paradigm for the early screening and diagnosis of autism spectrum disorders.

Additional features and advantages are described in and will be apparent from, the following Detailed Description and the Figures. The features and advantages described herein are not all-inclusive and, in particular, many additional features and advantages will be apparent to one of ordinary skill in the art in view of the figures and description. In addition, any particular embodiment does not have to have all of the advantages listed herein and it is expressly contemplated to claim individual advantageous embodiments separately. Moreover, it should be noted that the language used in the specification has been selected principally for readability and instructional purposes, and not to limit the scope of the inventive subject matter.

### BRIEF DESCRIPTION OF THE FIGURES

Fig. 1 shows receiver operating characteristics curve for the Autism Index (AI) predicting clinical Autism Spectrum Disorder (ASD) diagnosis (n=240), according to an example embodiment of the present disclosure.
Fig. 2 shows side-by-side histograms of Autism Index (AI) scores for Autism Spectrum Disorder (ASD) diagnosed and non-autistic control participants, according to an example embodiment of the present disclosure.
Fig. 3 shows a boxplot (+/- 95% CI) of Autism Index (AI) scores for Autism Spectrum Disorder (ASD) diagnosed and non-autistic control participants, according to an example embodiment of the present disclosure.
Fig. 4 shows a bivariate correlation between the Autism Index (AI) and Social Communication Questionnaire (SCQ) total raw scores, according to an example embodiment of the present disclosure.
Fig. 5 shows sample stimuli for the Qatari version (A and C) and Cleveland Clinic version (B and D) of an eye-tracking paradigm for the early screening and diagnosis of autism spectrum disorders, according to an example embodiment of the present disclosure.

### DETAILED DESCRIPTION

Example embodiments will now be described more fully with reference to the accompanying drawings.

Example embodiments are provided so that this disclosure will be thorough, and will fully convey the scope to those who are skilled in the art. Numerous specific details are set forth such as examples of specific components, devices, and methods, to provide a thorough understanding of embodiments of the present disclosure. It will be apparent to those skilled in the art that specific details need not be employed, that example embodiments may be embodied in many different forms and that neither should be construed to limit the scope of the disclosure. In some example embodiments, well-known processes, well-known device structures, and well-known technologies are not described in detail.

The terminology used herein is for the purpose of describing particular example embodiments only and is not intended to be limiting. As used herein, the singular forms "a," "an," and "the" may be intended to include the plural forms as well, unless the context clearly indicates otherwise. The terms "comprises," "comprising," "including," and "having," are inclusive and therefore specific the presence of stated features, integers, steps, operations, elements, and/or components, but do not preclude the presence or additional of one or more other features, integers, steps, operations, elements, components, and/or groups thereof. The method steps, processes, and operations described herein are not to be construed as necessarily requiring their performance in the particular order discussed or illustrated unless specifically identified as an order of performance. It is also to be understood that additional or alternative steps may be employed.

When an element or layer is referred to as being "on," "engaged to," "connected to," or "coupled to" another element or layer, it may be directly on, engaged, connected or coupled to the other element or layer, or intervening elements or layers may be present. In contrast, when an element is referred to as being "directly on," "directly engaged to," "directly connected to," or "directly coupled to" another element or layer, there may be no intervening elements or layers present. Other words used to describe the relationship between elements should be interpreted in a like fashion (e.g., "between" versus "directly between," "adjacent" versus "directly adjacent"). As used herein, the term "and/or" includes any and all combinations of one or more of the associated listed items.

Although the terms first, second, third, etc. may be used herein to describe various elements, components, regions, layers and/or sections, these elements, components, regions, layers and/or sections should not be limited by these terms. These terms may be only used to distinguish one element, component, region, layer or section from another region, layer or section. Terms such as "first," "second," and other numerical terms when used herein do not imply a sequence or order unless clearly indicated by the context. Thus, a first element, component, region, layer or section discussed below could be termed a second element, component, region, layer or section without departing from the teachings of the example embodiments.

Spatially relative terms, such as "inner," "outer," "beneath," "below," "lower," "above," "upper," and the like, may be used herein for ease of description to describe one element or feature's relationship to another element(s) or feature(s) as illustrated in the figures. Spatially relative terms may be intended to encompass different orientations of the device in use or operation in addition to the orientation depicted in the figures. For example, if the device in the figures is turned over, elements described as "below" or "beneath" other elements or features would then be oriented "above" the other elements or features. Thus, the example term "below" can encompass both an orientation of above and below. The device may be otherwise oriented (rotated 90 degrees or at other orientations) and the spatially relative descriptors used herein interpreted accordingly.

Methods, systems, and apparatus are disclosed herein for an Arabic language eye-tracking paradigm for the early screening and diagnosis of autism spectrum disorders.

While the example methods, apparatus, and systems are disclosed herein for an Arabic language eye-tracking paradigm for the early screening and diagnosis of autism spectrum disorders, it should be appreciated that the methods, apparatus, and systems may be operable for other applications.

Various abbreviations are used herein. Abbreviations: ASD = Autism Spectrum Disorder, DD = Developmentally Delay, NA = Non-Autistic, AI = Autism Index, AUC = Area Under Curve, ROC = Receiver Operating Characteristic, SCQ = Social Communication Questionnaire, ADOS-2 = Autism Diagnostic Observation Schedule - 2^{nd} edition.

The Disclosed Invention consists of a laptop computer, a desktop screen, and an eye tracker, like a Tobii tracker, that is connected to the desktop screen. The Disclosed Invention is simple and easily portable and only requires a designated room for administration. During the administration, the child either sits in the parent's lap or sits alone across from the screen. The examiner informs the child that he/she will be watching a short video and pictures, takes the child's information (name, age, code, prior diagnosis, date of examination), and then provides a countdown before the video begins. The child watches the videos while the examiner monitors the child's eye gaze on the screen to ensure that the data is being captured. Once finished, the data gets analyzed and provides a result of an Autism Index (AI) score.

The Disclosed Invention includes two modules of eye tracking stimuli paradigm to be used for the early screening and diagnosis of autism spectrum disorder as follows. Module one consists of 21 different photo and short video stimuli that the individual watches through a monitor with the eye tracking device attached to, and usually comes in two versions, Arabic and English. Module one is used for nonverbal and very young individuals. Module two consists of 24 different photo and short video stimuli that the individual watches through a monitor with the eye tracking device attached to, and usually comes in two versions, Arabic and English. Module two is used for verbal individuals.

A study was used for the creation and validation of the Disclosed Invention. The initial phase of the study included the translation of English language eye-tracking stimuli into stimuli appropriate for Arabic-speaking culture. During the second phase, the appropriateness of the stimuli was tested on 10 families (5 with ASD and 5 non-autistic controls).

All study participants were between the ages of 3-15 years. The study sample consisted of two groups: ASD (n=144) and control group (n=96), which consisted of NA (n=84), and DD (n=12). The ASD group was recruited from local special needs clinics and centers in which a prior ASD diagnosis was done. Cases suspected to be on the autism spectrum were also recruited and confirmed using the Disclosed Invention. The control group participants were either siblings of ASD participants, recruited through primary care clinics, or through research contacts. The total control sample consisted of 39% siblings of participants with ASD, and 61% were not related to any case of ASD. Given the small sample of DD, all control group participants were grouped into a single comparison sample. The exclusion criteria were for children with certain disabilities that will pose difficulties in performing the eye tracking testing (i.e. severe physical disabilities, visual and auditory impairments, etc.).

A diagnostic assessment was conducted for all ASD participants who never received a diagnosis using the ADOS-2, and for all participants suspected to be on the autism spectrum. A consensus diagnosis was based on a parent-interview, psychosocial, developmental and clinical history conducted by our research team whose members are all licensed administrators of the ADOS-2. After conducting the assessments, the presence/absence of ASD was confirmed using DSM-5 criteria (American Psychiatric Association, 2013). Eligibility for participation in the DD group required any other neurodevelopmental or neuropsychiatric disorder diagnosis other than ASD. Eligibility in the NA group required no past or current developmental or psychiatric difficulties. All group participants were screened using the SCQ, and seen by a neurodevelopmental physician with years of experience in ASD phenotyping and diagnosis, to ascertain or rule out ASD in each sample.

The clinical assessments in the research protocol included the ADOS-2, Arabic, and English versions of the SCQ and DSM-5 criteria. As the gold-standard clinical observation measure used to assess autism symptoms severity, the ADOS-2 was used. In the study, the ADOS-2 total, social affect sub-scale, and restricted/repetitive behavior sub-scale raw scores were converted to calibrated severity scores based on the ADOS-2 module used and the comparison scores for each participant. During a parent interview, the research team completed the Arabic or English version of the SCQ and a clinical and developmental history form. As part of clinical history, results from previous assessments such as IQ, language, and other developmental concerns, were requested from the families of consented participants and retrieved from clinical history records of record-review participants.

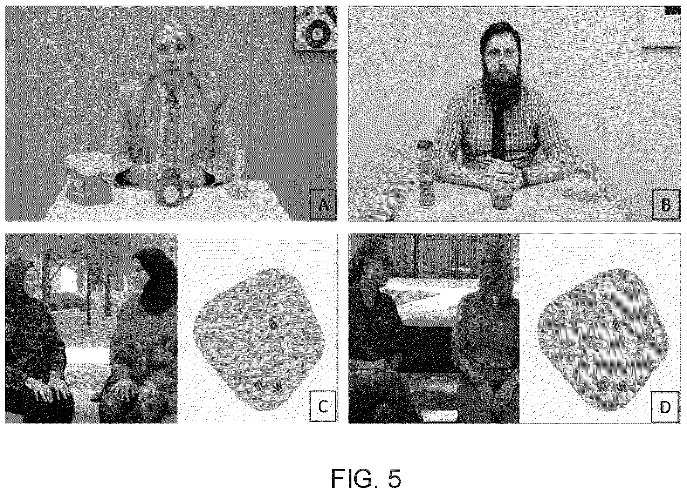

The creation of the Arabic version of the eye tracking stimuli was completed in three stages. First stage was obtaining the English version (Cleveland Clinic) of the eye tracking stimuli. Second stage included the translation and back translation (from English to Arabic) of the content by the QBRI team. Lastly, a local Qatari production company was hired for the creation of the Arabic version of the eye tracking stimuli. The research team replicated the setup and setting of the English version at Hamad Bin Khalifa University (HBKU). The team then reviewed the content, compared them to match the Cleveland Clinic (CC) stimuli as much as possible, and recommended adjustments as needed. ROIs were identified in the Qatari Stimuli to match the English Stimuli. Figure 5 shows sample stimuli for the Qatari version (A and C) and Cleveland Clinic version (B and D).

Eye tracking data was collected in a quiet room, using the SMI RED250 remote eye tracker system attached to a 19-inch LCD stimulus presentation monitor. Binocular gaze, 3D eye position, pupil, and timestamp data were collected at a sampling rate of 250Hz. Gaze capture was automatically calibrated using 2-, 5-, or 9-point calibrations (starting with 5 or 9-point depending on functional level of the child) and provided position accuracy to 0.4°. Dwell proportion (percentage on target region relative to total time on screen) was the primary measurement of interest, although additional measures were also collected, including visits to regions-of-interest, saccade average length, saccade peak velocity, and blink frequency.

For the eye tracking assessment, the child was seated alone or in his/her parent's lap approximately 60-65 cm from the LCD display and viewed the stimuli subtending a visual angle of approximately 18.8 degrees. Standard room lighting was used, and the room was sparse, with visual barriers to reduce distraction. After calibration, children were told, "You will see some videos, pay attention, but look however you want". Stimuli was presented using the SMI Experiment Center. Gaze data was captured during viewing of a 10 min battery consisting of initial and recurring calibration and multiple stimuli from each of the following paradigms: dynamic individual faces, static side-by-side faces, joint attention bids, gaze following, reciprocal interactions, dynamic social versus dynamic geometric images, and passive viewing of social/object arrays. The visual paradigm ended with a short gaze accuracy validation step. The same measurement techniques were used in the study as in the previous, English-language study, but the study calculated z-scores using the mean and standard deviation of the English-language study. The indicators that showed less attention to social cues in children with ASD (which were given a negative value) were combined with indicators that showed more attention to non-social cues in ASD-affected individuals. Test-retest comparisons were done for 28 participants from all groups, the time between administrations of the test and retest averaged 8 months (Table 6).

The study identified outliers and high leverage cases using univariate and bivariate distributions. Analyses were performed with and without these cases, but there were no significant differences, so all available data was included. Descriptive statistics were used to characterize the sample and comparisons were made between baseline and retest participants using appropriate statistical tests. Receiver operating characteristic (ROC) curve analysis assessed the validity of the AI, SCQ, and ADOS-2 severity scores. Concurrent validity for autism severity was evaluated using Spearman's rank-order correlations between these measures. However, the correlation between ADOS-2 scores and other measures was anticipated to be lower due to the restricted range of these scores. The internal consistency reliability of the AI and its attention indicators was estimated using Cronbach's α, while test-retest reliability was evaluated using Pearson's r. Area under the ROC curve was used to quantify validity. To establish diagnostic validity, a 95% confidence interval of the ROC curve should be larger than .80.

To validate the Arabic language AI, the study computed Spearman rank-order correlation coefficients examining the relationships between the social vs. non-social attention indices, the ADOS-2 total, severity scores, and SCQ raw scores. ADOS-2 calibrated severity scores were computed using existing norms. A type 1 error rate of .05 was used for each analysis. In addition, to avoid inflation of Type 1 error for these correlations, the study used a Benjamini-Hochberg false discovery rate correction and only correlations exceeding r=.40 were considered clinically meaningful. The validation sample was intentionally over-powered for detection of a significant area under the ROC curve (AUC of >.70). Descriptive statistics were used to characterize the sample and comparisons were made between baseline and retest participants using Chi-square (categorical variables) or t-tests (continuous variables) and Cohen's d was presented to evaluate the magnitude of baseline and retest sample differences.

Overall study results: The AI had excellent internal consistency and test-retest reliability. Moreover, the AI showed good differentiation of ASD from control cases (AUC=.730, SE=.035). The AI was significantly positively correlated with SCQ total raw scores (r=.46, p<.001). ADOS-2 scores were only available in the ASD group and did not show a significant relationship with AI scores (r=.10, p=.348), likely due to restricted range.

The final baseline sample included 240 participants (144 ASD, 84 NA, and 12 DD) and the retest sample included 28 participants (16 ASD, 9 NA, and 3 DD) (Table 1). The distribution of diagnoses, age, sex, ASD sex ratio, SCQ total raw scores, tracking ratio, and number of valid stimuli were consistent between the baseline sample and the retest sample. However, the retest sample had significantly higher proportions of participants with language/communication disorders and global developmental delay/intellectual disability. Overall, both the baseline and retest samples consisted of individuals with wide ranges of ages, neuropsychiatric diagnoses, and autism symptoms, making the present sample a relatively challenging case for diagnostic differentiation.

**Table 1: Participant characteristics in the full baseline sample and the retest sub-sample.**

| | | Baseline M (SD) | Retest M (SD) | X²/t (p) | Cohen's d |
|---|---|---|---|---|---|
| Total N | | 240 | 28 | 2.15 (.342) | .19 |
| | Non-Autistic (n, %) | 84 (35.0%) | 9 (32.1%) | | |
| | Developmental Delay (n, %) | 12 (5.0%) | 3 (10.7%) | | |
| | Autism Spectrum Disorder (n, %) | 144 (60.0%) | 16 (57.2%) | | |
| Age (range) | | 7.7 (3.6, 1.4-16) | 7.7 (3.1, 1.4-13) | -0.80 (.935) | 0.10 |
| Female (n, %) | | 75 (31.3%) | 8 (28.6%) | 0.13 (.721) | 0.05 |
| ASD Sex Ratio (female:male) | | 1:3.7 | 1:4.3 | 0.10 (.751) | 0.04 |
| Other diagnoses (n, %) | | | | 19.6 (.001) | 0.60 |
| | Language or Communication Disorder | 51 (21.2%) | 16 (57%) | | -4.18 |
| | GDD/ID | 19 (7.9%) | 8 (28.6%) | | -3.45 |
| | Anxiety Disorder | 28 (11.7) | 1 (3.6%) | | 1.30 |
| | Attention-Deficit/Hyperactivity Disorder | 86 (35.8%) | 6 (21.4%) | | 1.52 |
| | Other | 19 (7.9%) | 1 (3.6%) | | 0.82 |
| *SCQ* Total Raw Score | | | | | |
| | Non-Autistic | 1.4 (2.2) | 0.8 (0.4) | 0.92 (.363) | 12 |
| | Developmental Delay | 4.0 (4.9) | - | - | |
| | Autism Spectrum Disorder | 16.4 (7.2) | 20.1 (5.1) | -1.97 (.051) | .26 |
| *ADOS-2* Total Severity (Autism cases only) | | 6.1 (2.1) | - | - | |
| Overall Tracking Ratio (%) | | 79.1% (14.3%) | 77.6% (15.2%) | 0.64 (.526) | 08 |
| Number of Valid Stimuli (out of 44) | | 35.3 (7.9) | 34.3 (8.1) | 0.75 (.457) | 10 |

Note. DD= developmental delay. SCQ=Social Communication total raw score Chi-square statistics were converted to Cramer's V. As an effect size metric, Cramer's V is roughly equivalent to r and, therefore, to provide a common metric, Cramer's V was converted to Cohen's d via r. Only one ADOS-2 Total Severity score was available in the retest sample and no SCQ total raw scores were available in the Developmental Delay group for the retest sample.

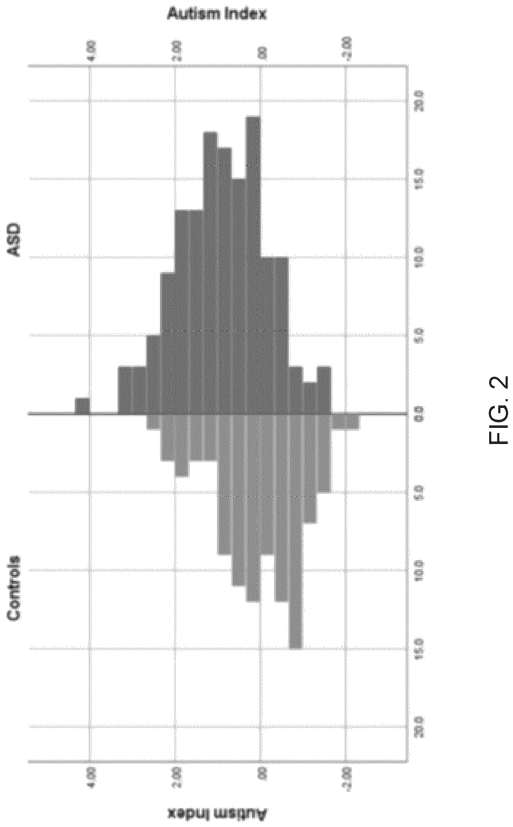

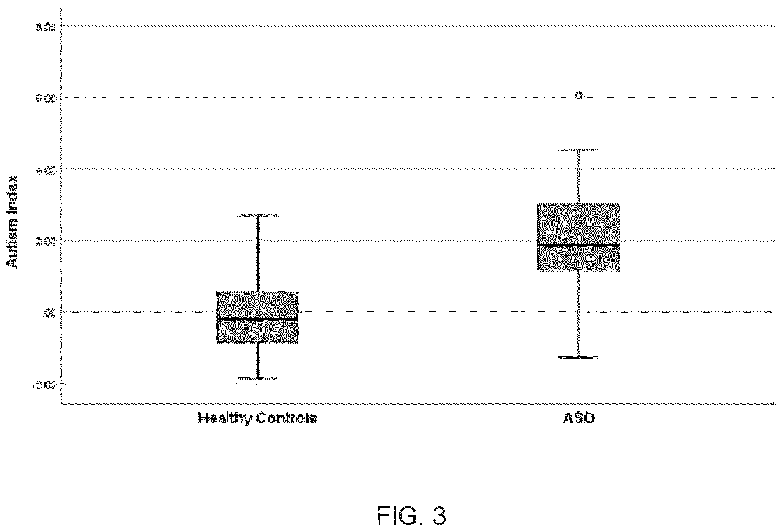

Autism Index (AI) Score Range and Reliability: Figure 2 shows side-by-side histograms of Autism Index (AI) scores for ASD diagnosed and non-autistic control participants and Figure 3 shows a boxplot (+/- 95% CI) of Autism Index (AI) scores for ASD diagnosed and non-autistic control participants. As seen in Figures 2 and 3, AI scores were fairly normally distributed in the ASD (skew=0.21, kurtosis=0.08) and control (skew=0.34, kurtosis=-0.40) participants showed a wide range of scores on the AI (z=-2.28 to +4.26) with a pronounced shift upward in the ASD group (Cohen's d=0.86) (see also Table 1). When considered separately, the social and non-social attention indicators had very good to excellent internal consistency and test-retest reliability. Internal consistency and test-retest reliability was excellent (see Table 2).

**Table 2: Internal consistency and test-retest reliability coefficients for social and non-social attention indicators and the AI (k=number of gaze indicators).**

| | Internal Consistency α | Test-Retest r |
|---|---|---|
| Social Attention (k=177) | .90 | .73 |
| Non-Social Attention (k=195) | .80 | .44 |
| Autism Index (k=372) | .91 | .73 |

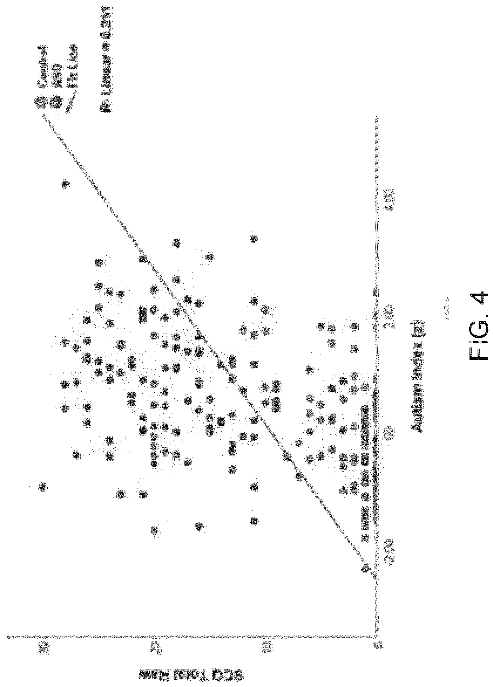

Diagnostic and Concurrent Validity: Figure 1 shows receiver operating characteristics curve for the Autism Index (AI) predicting clinical Autism Spectrum Disorder (ASD) diagnosis (n=240). As seen in Figure 1, AI scores showed good differentiation of ASD from control cases (AUC=.730, SE=.035). Evaluating subsets of cases with more stringent validity (Tracking ratio≥.80, number of valid stimuli ≥35) did not improve diagnostic accuracy (AUC=.689, SE=.050) likely due to loss of more significantly affected ASD cases. In the full sample, AI scores were significantly positively correlated with SCQ total raw scores (r=.46, p<.001), as seen in Figure 4, which shows the bivariate correlation between the AI and Social Communication Questionnaire (SCQ) total raw scores. ADOS-2 scores were only available in the ASD group and these scores did not show a significant relationship with AI scores (r=. 10, p=.348), likely due to somewhat restricted range of scores in this group (82.6% of scores fell between 3-8).

The observed AI diagnostic accuracy is impressive given that the original index selected a subset of social and non-social indicators showing significant but modest validity for ASD diagnosis and conducted linear averaging across these indicators. For the present study, this average was rigidly applied to examine strict replicability and extension to a Qatar population. Thus, the observed diagnostic accuracy is likely conservative, as one would expect that a more optimal set of indicators and algorithm might be obtained. To explore this possibility, the available indicators used to compute the AI were input to support vector machine and Random Forest analyses to predict ASD diagnosis. Results indicated a potential improvement in validity, using the same indicator set, by applying a support vector machine (radial basis kernel, 230 support vectors, cost=1, gamma=.003, epsilon=0.1, 10-fold cross-validation) or a random forest algorithm (500 trees, variables tried at each split=123, leave-one-out cross-validation) (SVM - R2=.26, AUC=.799; RF - R2=.21, AUC=.768).

The AI, when implemented using Arabic-translated stimuli in a Qatari sample, ("the Arabic AI") showed good diagnostic differentiation and a strong correlation with parent-reported ASD symptoms. Thus, the AI appears to have cross-cultural validity and may be useful as a diagnostic aide to inform clinical judgment and track ASD symptom levels as part of the evaluation process.

Results indicated that the Arabic AI showed good diagnostic differentiation, which supports its utility for screening and as a diagnostic aide to inform clinical judgment. These findings are consistent with previous studies that have reported the efficacy of the English version of the AI or similar algorithms in identifying children at risk for ASD. The study also found a strong correlation between the Arabic AI and the SCQ, which suggests its ability to discriminate between symptom levels in children with ASD. These findings are in line with previous studies that have demonstrated the validity of the SCQ as a screening tool for ASD.

Moreover, the high internal consistency and test-retest reliability of the Arabic AI in the study supports its potential clinical value as an assessment tool for ASD. Prior studies on gaze-based measures have suggested moderate test-retest stability. However, the study found strong test-retest reliability for the social attention indicators and moderate reliability for the non-social indicators, which suggest that the Arabic AI is measuring a stable trait consistent with the diagnosis of ASD. These findings are consistent with previous studies that have reported the stability of gaze-based measures in children with ASD.

Objective measures are necessary to grade the severity of ASD symptoms and monitor changes over time, as subjective measures may be inconsistent and often require substantial training and ongoing reliability checks to maintain accuracy. The results of the study suggest that the Arabic AI may be a useful tool in conjunction with other commonly used clinical measures for identifying ASD. Interestingly, supporting the contention of incremental validity with other clinical measures, missed cases also had a different pattern of ADOS-2 scores compared to correctly identified cases.

**Table 3: Sample demographics and eye tracking quality for valid and invalid participants. Note. Invalid cases were individuals with fewer than 15 valid stimuli out of a possible 40. All participants (valid and invalid) had tracking ratios above 40%. The ADOS-2 was only administered to ASD-affected individuals and a small number of NA controls where concern of ASD was identified but ruled out. SCQ scores were only available for 107 of 155 participants.**

| | Valid M (SD) | Invalid M (SD) | X², t (p) |
|---|---|---|---|
| N | 136 | 19 | |
| Age | 7.6 (3.4) | 6.5 (4.0) | 1.34 (.182) |
| Male (N, %) | 92 (67.6%) | 8 (42.1%) | 4.75 (.029) |
| SCQ | 9.9 (9.2) | 18.9 (7.8) | 2.81 (.006) |
| ADOS Raw | 162 (5.1) | 19.8 (3.6) | 2.03 (.046) |
| ADOS Total Calibrated Severity Score | 6.1 (1.9) | 6.7 (1.4) | 0.89 (.378) |
| ASD (n, %) | 85 (62.5%) | 14 (73.7%) | 0.90 (.342) |
| Number of Valid Stimuli | 31.5 (7.2) | 10.5 (2.9) | 12.53 (<.001) |
| Tracking Ratio (%) | 80.1% (13.2%) | 48.0% (6.3%) | 10.38 (<.001) |

**Table 4: Sample demographics and eye tracking quality for ASD diagnosed and non-autistic control participants.**

| Note. Invalid cases were individuals with fewer than 15 valid stimuli out of a possible 40. All participants (valid and invalid) had tracking ratios above 40%. The ADOS was only administered to ASD-affected individuals and a small number of non-autistic controls where concern of ASD was identified but ruled out. SCQ scores were only available for 107 of 155 participants. | | | |
|---|---|---|---|
| | ASD M (SD) | Non-Autistic Controls M (SD) | X2,t(p) |
| N | 85 | 51 | |
| Age | 8.5 (3.1) | 6.1 (3.3) | 4.32 (<.001) |
| Male (N, %) | 70 (82.4%) | 22 (43.1%) | 22.40 (<.001) |
| SCQ | 15.9 (7.7) | 1.7 (2.3) | 11.41 (<.001) |
| ADOS Raw | 16.2 (5.1) | | |
| ADOS Total Calibrated Severity Score | 6.1 (1.9) | | |
| Number of Valid Stimuli | 29.0 (7.0) | 35.7 (5.5) | 5.82 (<.001) |
| Tracking Ratio (%) | 75.1% (12.5%) | 88.4% (10.0%) | 6.47 (<.001) |

**Table 5: Summary Statistics of the Test-Retest samples.**

| | |
|---|---|
| Mean (Average) | 8.1921875 |
| Median | 8.18 |
| Range | 5.5 |
| Geometric Mean | 8.069696 |
| Standard Deviation | 1.3561866 |
| Variance | 1.83924209 |
| Sample Standard Deviation | 1.37788696 |
| Sample Variance | 1.89857248 |

**Table 6: Test-Retest Samples, details of period between tests.**

| **TEST** | **RETEST** | **NUMBER OF DAYS** | **NUMBER OF MONTHS** |
|---|---|---|---|
| **5/14/2019** | 10/14/2019 | 153 | 5 |
| **3/24/2019** | 12/9/2019 | 260 | 8.15 |
| **3/25/2019** | 12/9/2019 | 259 | 8.14 |
| **3/25/2019** | 12/5/2019 | 255 | 8.10 |
| **3/25/2019** | 12/10/2019 | 260 | 8.15 |
| **3/25/2019** | 12/11/2019 | 261 | 8.16 |
| **3/27/2019** | 12/8/2019 | 256 | 8.11 |
| **4/4/2019** | 12/5/2019 | 245 | 8.1 |
| **3/28/2019** | 12/10/2019 | 257 | 8.12 |
| **3/28/2019** | 12/10/2019 | 257 | 8.12 |
| **3/28/2019** | 12/11/2019 | 258 | 8.13 |
| **3/28/2019** | 12/9/2019 | 256 | 8.11 |
| **4/1/2019** | 12/9/2019 | 252 | 8.8 |
| **4/1/2019** | 12/8/2019 | 251 | 8.7 |
| **4/2/2019** | 12/8/2019 | 250 | 8.6 |
| **4/3/2019** | 12/11/2019 | 252 | 8.8 |
| **4/3/2019** | 12/12/2019 | 253 | 8.9 |
| **4/3/2019** | 12/11/2019 | 252 | 8.8 |
| **4/3/2019** | 12/9/2019 | 250 | 8.6 |
| **4/3/2019** | 12/5/2019 | 246 | 8.2 |
| **8/4/2019** | 1/13/2020 | 162 | 5.9 |
| **8/4/2019** | 1/13/2020 | 162 | 5.9 |
| **8/4/2019** | 1/13/2020 | 162 | 5.9 |
| **8/4/2019** | 1/13/2020 | 162 | 5.9 |
| **2/25/2019** | 1/12/2020 | 321 | 10.18 |
| **2/25/2019** | 1/12/2020 | 321 | 10.18 |
| **2/25/2019** | 1/12/2020 | 321 | 10.18 |
| **3/7/2019** | 1/12/2020 | 311 | 10.5 |
| **3/7/2019** | 1/12/2020 | 311 | 10.5 |
| **4/13/2019** | 1/12/2020 | 274 | 8.30 |
| **4/13/2019** | 1/12/2020 | 274 | 8.30 |
| **4/17/2019** | 1/13/2020 | 271 | 8.27 |
| **7/7/2019** | 1/12/2020 | 189 | 6.5 |

The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the disclosure, and all such modifications are intended to be included within the scope of the disclosure.

## Claims

1. A system for screening and diagnosis of autism spectrum disorders using an Arabic language eye-tracking paradigm, comprising:
a computing device;
a screen device;
an eye tracking device; and
a set of stimuli paradigm, wherein the set of stimuli paradigm are each comprised of dynamic individual faces, static side-by-side faces, joint attention bids, gaze following, reciprocal interactions, dynamic social versus dynamic geometric images, and passive viewing of social or object arrays, and wherein the set of stimuli paradigm each end a short gaze accuracy validation step.

2. The system of Claim 1, wherein the computing device is operatively coupled to the screen device and the eye tracking device.

3. The system of Claim 2, wherein the eye tracker is operatively coupled to the screen device, such that the eye tracker tracks individual eye gaze information for an individual looking at the screen device.

4. The system of Claim 3, wherein the individual eye gaze information tracked is comprised of binocular gaze, 3D eye position, pupil, and timestamp data collected at a sampling rate of 250Hz, wherein the individual eye gaze information tracked is calibrated using 2-, 5-, or 9-point calibrations starting with 5 or 9-point depending on a functional level of the individual looking at the screen device, and wherein the individual eye gaze information tracked is provided with position accuracy to 0.4°.

5. The system of Claim 4, wherein the computing device is configured to play one of the set of stimuli paradigm.

6. The system of Claim 5, wherein the computing device is configured to calculate dwell proportion based on the individual eye gaze information tracked, wherein dwell proportion is a percentage of on target region relative to total time on screen of the one of the set of stimuli paradigm played, and wherein the computing device is configured to collect information on visits to regions-of-interest, saccade average length, saccade peak velocity, and blink frequency based on the individual eye gaze information tracked.

7. The system of Claim 6, wherein the computing device is configured to calculate an Autism Index (AI) score.

8. The system of Claim 1, wherein the set of stimuli paradigm is comprised of a first module and a second module.

9. The system of Claim 8, wherein the first module is comprised of 21 different photo and short video stimuli entirely in Arabic or entirely in English, and wherein the first module is configured for use with nonverbal and very young individuals.

10. The system of Claim 8, wherein the second module is comprised of 24 different photo and short video stimuli entirely in Arabic or entirely in English, and wherein the second module is configured for use with verbal individuals.

11. The system of Claim 1, wherein the eye tracking device is one of a Tobbi or and SMI RED250 eye tracker system.

12. A method of using a system for screening and diagnosis of autism spectrum disorders using Arabic language eye-tracking paradigm comprising a computing device, a screen device, an eye tracking device and a set of stimuli paradigm, wherein the set of stimuli paradigm are each comprised of dynamic individual faces, static side-by-side faces, joint attention bids, gaze following, reciprocal interactions, dynamic social versus dynamic geometric images, and passive viewing of social or object arrays, and wherein the set of stimuli paradigm each end a short gaze accuracy validation step, the method comprising:
playing one of the set of stimuli on the screen device;
tracking individual eye gaze information for an individual looking at the screen device; and
calculating an Autism Index (AI) score based on the individual eye gaze information.

13. The method of Claim 12, wherein the computing device is operatively coupled to the screen device and the eye tracking device, and wherein the eye tracker is operatively coupled to the screen device, such that the eye tracker is configured to track individual eye gaze information for the individual looking at the screen device.

14. The method of Claim 12, wherein the individual eye gaze information tracked is comprised of binocular gaze, 3D eye position, pupil, and timestamp data collected at a sampling rate of 250Hz, wherein the individual eye gaze information tracked is calibrated using 2-, 5-, or 9-point calibrations starting with 5 or 9-point depending on a functional level of the individual looking at the screen device, and wherein the individual eye gaze information tracked is provided with position accuracy to 0.4°.

15. The method of Claim 12, wherein the computing device is configured to calculate dwell proportion based on the individual eye gaze information tracked, wherein dwell proportion is a percentage of on target region relative to total time on screen of the one of the set of stimuli paradigm played, and wherein the computing device is configured to collect information on visits to regions-of-interest, saccade average length, saccade peak velocity, and blink frequency based on the individual eye gaze information tracked.

16. The method of Claim 12, wherein the set of stimuli paradigm is comprised of a first module and a second module.

17. The method of Claim 16, wherein the first module is comprised of 21 different photo and short video stimuli entirely in Arabic or entirely in English, wherein the first module is configured for use with nonverbal and very young individuals, wherein the second module is comprised of 24 different photo and short video stimuli entirely in Arabic or entirely in English, and wherein the second module is configured for use with verbal individuals.

18. The method of Claim 12, wherein the individual looking at the screen device is located about 60 to 65 cm from the screen device, and wherein the individual looking at the screen device looks at the screen device at a visual angle of about 18.8 degrees.

19. The method of Claim 12, wherein the individual eye gaze information tracked is tracked for 3-5 minutes.

20. A method of calculating an Autism Index (AI) score, the method comprising:
collecting individual eye gaze information for an individual looking at one of a set of stimuli paradigm;
collecting information on visits to regions-of-interest, saccade average length, saccade peak velocity, and blink frequency based on the individual eye gaze information; and
calculating a dwell proportion based on the individual eye gaze information;
wherein the set of stimuli paradigm is comprised of a first module and a second module;
wherein the first module is comprised of 21 different photo and short video stimuli entirely in Arabic or entirely in English;
wherein the first module is configured for use with nonverbal and very young individuals;
wherein the second module is comprised of 24 different photo and short video stimuli entirely in Arabic or entirely in English;
wherein the second module is configured for use with verbal individuals; wherein the individual eye gaze information tracked is comprised of binocular gaze, 3D eye position, pupil, and timestamp data collected at a sampling rate of 250Hz;
wherein the individual eye gaze information tracked is calibrated using 2-, 5-, or 9-point calibrations starting with 5 or 9-point depending on a functional level of the individual looking at the screen device;
wherein the individual eye gaze information tracked is provided with position accuracy to 0.4°; and
wherein dwell proportion is a percentage of on target region relative to total time on screen of the one of the set of stimuli paradigm played.
